# FASCICULE DE BREVET EUROPEEN

(11) **EP 4 640 671 B1**
(45) Date de publication et mention de la délivrance du brevet: **18.11.2026**
(21) Numéro de dépôt: 25172154.4
(22) Date de dépôt: 24.04.2025
(51) Int. Cl.: C07D 207/27, C08F 22/36

(54) **PROCÉDÉ DE SYNTHÈSE D'AGENTS DE LIAISON POLYMÈRE NEUTRES**
VERFAHREN ZUR SYNTHESE NEUTRALER POLYMERBINDEMITTEL
PROCESS FOR THE SYNTHESIS OF NEUTRAL POLYMER BINDERS

(30) Priorité: 26.04.2024 FR 2404267
(43) Date de publication de la demande: 29.10.2025
(73) Titulaire: ARIANEGROUP SAS, 78130 Les Mureaux (FR)
(72) Inventeur: EYMANN, John, 91710 VERT LE PETIT (FR); QUINTARD, Emilie, 91710 VERT LE PETIT (FR); VANDECANDELAERE, Nicolas, 91710 VERT LE PETIT (FR); COMTE, Sébastien, 91710 VERT LE PETIT (FR)
(74) Mandataire: Cabinet Beau de Loménie

(56) Documents cités:
- CN-A- 115 626 969
- US-A- 4 915 755
- SONG JUNMING ED - GUIYANG ET AL: "Research on the Synthesis and Performance of a New Type of Neutral Polymer Bonding Agent", JOURNAL OF PHYSICS: CONFERENCE SERIES, INSTITUTE OF PHYSICS PUBLISHING, GB, vol. 2152, no. 1, 1 December 2021 (2021-12-01), XP020413404, ISSN: 1742-6588, [retrieved on 20211201], DOI: 10.1088/1742-6596/2152/1/012004
- ZHOU ET AL., IRANIAN POLYMER JOURNAL, vol. 28, no. 11, 2019, pages 943 - 955, XP036922634

## Description

### Domaine technique de l'invention

La présente divulgation concerne le domaine des matériaux énergétiques, et plus particulièrement un procédé de synthèse de composés intermédiaires utiles dans la préparation desdits matériaux.

### Etat de la technique

Les propergols solides de type composite ont un large éventail d'applications notamment dans le secteur de la propulsion. Ces propergols sont typiquement préparés à partir d'un mélange de combustible, d'oxydant et de liant polymère. Une petite quantité d'agent de liaison peut y être incorporée afin de garantir les propriétés mécaniques de ces matériaux. Des agents de liaison de type « NPBA » (acronyme de l'anglais Neutral Polymeric Binding Agents ») sont décrits dans le brevet US 4915755, par exemple le polymère de formule : 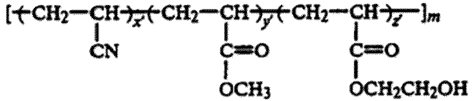 dans laquelle x' vaut 1, y' est dans la plage 0-0,5 et z' est dans la plage 0.1-0.5. Plus récemment, Zhou et al. (Iranian Polymer Journal, vol. 28, n° 11, pp. 943-955, 2019) ont décrit la synthèse de NPBA à partir de toluène diisocyanate et d'éthoxylate de propynol. Toutefois, l'utilisation de diisocyanates n'est pas souhaitable en raison de la dangerosité de ces produits et de leur mise en œuvre.

Dans ce contexte, les inventeurs se sont fixés comme cahier des charges la synthèse d'agents de liaison polymères neutres qui satisfont à la règlementation REACH et qui sont compatibles avec une utilisation dans des matériaux énergétiques.

### Résumé de l'invention

La présente divulgation concerne un procédé de synthèse d'un composé de formule (I) : 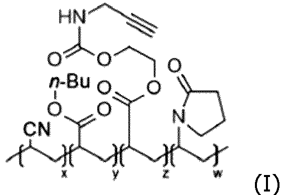
dans laquelle x = 1 ; 0 < w ≤ 1 ; 0 < y ≤ 1 et 0 < z ≤ 1 ;
le procédé comprenant :
   a) la réaction d'un composé de formule (II) : 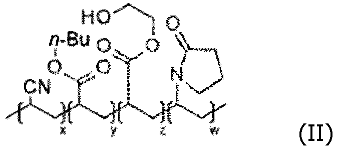
      dans laquelle, w, x, y et z sont tels que définis ci-dessus pour la formule (I),
      avec un excès de carbonyldiimidazole, éventuellement en présence d'un solvant ;
   b) la réaction du composé obtenu à l'étape a) avec un excès de propargylamine, éventuellement en présence d'un solvant.

La présente divulgation concerne également les composés de formule (I) susceptibles d'être obtenus par ledit procédé.

### Description de l'invention

Dans le contexte de la présente divulgation, l'expression "compris entre x et y" doit être interprétée comme incluant les bornes de la plage considérée (i.e., x et y).

Les différents modes de réalisation décrits ici peuvent être combinés.

Selon un aspect, la présente divulgation concerne un procédé de synthèse d'un composé de formule (I) : 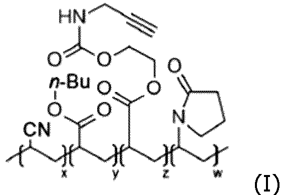 dans laquelle x = 1 ; 0 < w ≤ 1 ; 0 < y ≤ 1 et 0 < z ≤ 1 ; le procédé comprenant :
a) la réaction d'un composé de formule (II) : 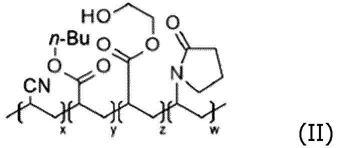 dans laquelle, w, x, y et z sont tels que définis ci-dessus pour la formule (I),
   avec un excès de carbonyldiimidazole, éventuellement en présence d'un solvant ;
b) la réaction du composé obtenu à l'étape a) avec un excès de propargylamine, éventuellement en présence d'un solvant.

L'étape a) est mise en œuvre en présence d'un excès de carbonyldiimidazole. Par « excès de carbonyldiimidazole » on entend une quantité comprise entre environ 1,1 et environ 6 équivalents.

Les composés de formule (II) peuvent être obtenus par exemple comme décrit dans le brevet US 4915755.

Le composé obtenu à l'issue de l'étape a) est un composé « intermédiaire » de formule : 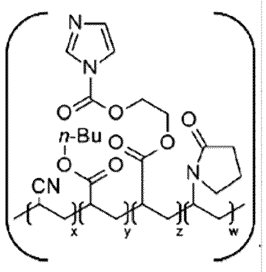 dans laquelle, w, x, y et z sont tels que définis ci-dessus pour la formule (I), composé qui est utilisé tel quel pour la mise en œuvre de l'étape b).

L'étape b) est mise en œuvre en présence d'un excès de propargylamine. Par « excès de propargylamine» on entend une quantité comprise entre environ 1,5 et environ 6 équivalents.

Dans certains modes de réalisation, l'étape a) est mise en œuvre à une température comprise entre la température ambiante et environ 40°C, avantageusement à une température comprise entre la température ambiante et environ 30°C.

Dans certains modes de réalisation, la durée de l'étape a) est comprise entre environ 15 minutes et environ 10h, avantageusement entre environ 15 minutes et environ 3h.

Dans certains modes de réalisation, l'étape b) est mise en œuvre à une température comprise entre la température ambiante et environ 40°C, avantageusement à une température comprise entre la température ambiante et environ 30°C. Dans certains modes de réalisation, la durée de l'étape b) est comprise entre environ 1h et environ 48h, avantageusement entre 1h et environ 24h.

Dans certains modes de réalisation, l'étape a) est mise en œuvre en présence d'un solvant. En pratique, il est possible d'utiliser un solvant organique usuel, à l'exception des solvants nucléophiles. Avantageusement, le solvant peut être choisi parmi le chlorure de méthylène, le chloroforme, le 1,2-dichloroéthane, l'acétate d'éthyle.

Dans certains modes de réalisation, l'étape b) est mise en œuvre en présence d'un solvant. En pratique, il est possible d'utiliser un solvant organique usuel, à l'exception des solvants nucléophiles. Avantageusement, le solvant peut être choisi parmi le chlorure de méthylène, le chloroforme, le 1,2-dichloroéthane, l'acétate d'éthyle.

Dans certains modes de réalisation, le solvant utilisé à l'étape a) et le solvant utilisé à l'étape b) sont identiques.

Le procédé selon la présente divulgation présente les avantages suivants :
- c'est une synthèse « one-pot » ;
- il donne des rendements quantitatifs ;
- il met en œuvre des réactifs peu toxiques et bon marché ;
- il permet une purification aisée du produit final.

Selon un autre aspect, la présente divulgation concerne les composés de formule (I) susceptibles d'être obtenus par le procédé décrit ci-dessus. Ces composés sont utiles comme agents de liaison polymères neutres pour la préparation de matériaux énergétiques tels que des propergols solides composites.

L'invention sera mieux comprise à l'aide de l'exemple suivant, donné à titre illustratif. Dans cet exemple, la masse molaire moyenne en nombre (Mn) et en masse (Mp) du composé de formule (I) a été déterminée par chromatographie par exclusion stérique (SEC) à l'aide de l'appareil suivant :
- Equipement : Agilent Infinity 1260 thermostatée à 70°C ;
   - Détection: réfractométrie;
- Eluant: Diméthylsulfoxide, 1,0 ml/min ;
   - Etalon: Polyméthacrylate de méthyle.

La polydispersité Ð du composé de formule (I) est égale au rapport Mp/Mn.

Le composé de formule (I) a par ailleurs été caractérisé par RMN à l'aide d'un appareil Bruker AC-400.

### Abréviations

AIBN = azobisisobutyronitrile
CDI = carbonyldiimidazole
DCM = dichlorométhane

### Exemple 1

### Synthèse d'un composé de formule :

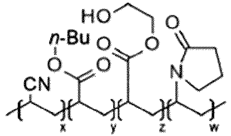

dans laquelle x = 1; y = 0,3; z = 0,2; w = 0,1

Un ballon tricol de 100mL est équipé d'un thermomètre, d'un réfrigérant et d'une agitation mécanique (300 tr/min). Il a ensuite été chargé avec 6 mL d'acétone et plongé dans un bain-marie à température ambiante. L'acrylonitrile (4 mL, 60,3 mmoles), l'acrylate de *n*-butyle (2,58 mL, 18,1 mmoles), l'acrylate de 2-hydroxyéthyle (1,39 mL, 12,1 mmoles), la N-vinylpyrrolidone (640 µL, 6,0 mmoles) et le mercaptoéthanol (240 µL, 3,38 mmoles) ont été additionnés par portions à l'aide de seringues. L'AIBN (206,38 mg, 1,26 mmoles, 1,3 mole%) a ensuite été ajouté et les résidus rincés avec 1 mL supplémentaire d'acétone. La température de consigne du bain-marie a ensuite été fixée à 64°C. La réaction s'est poursuivie durant 6h à 60°C, puis du méthanol (20 mL) a été additionné pour stopper la réaction. Le milieu a ensuite été récupéré avec de l'acétone et évaporé intégralement sous pression réduite afin de ne garder que le copolymère. Ce dernier a ensuite été séché toute une nuit sous vide profond (10⁻² mbar) à température ambiante. On a obtenu un solide jaune (6,88 g, 85%).

**¹H RMN** (acétone d₆, 400 MHz, 21°C): δ (ppm) = 4.23 (s(br), -OC***H₂***CH₂OH), 4.14 (s(br), -OCH***H₂***H₂CH₂CH₃), 3.78 (s(br), -OCH₂C***H₂***OH), 3.58 - 2.47 (m, C***H_{chaîne principale}***)*,* 2.45 - 1.72 (m, C***H_{2 chaîne principale}***)*,* 1.58 (s(br), -OCH₂C***H₂***CH₂CH₃), 1.35 (s(br), -OCH₂CH₂C***H₂***CH₃), 0.88 (s(br), -OCH₂CH₂CH₂C***H₃***).

**ATR-FTIR** : ν (cm⁻¹) = 3505 (w(b)), 2959 (m), 2935 (m), 2874(w), 2242(w), 1725(s), 1670(m), 1452(m), 1267(m), 1226(m), 1166(s), 1067(m), 936(w), 891(w), 842(w), 739(w), 652(w).

**SEC** (DMSO, Indice de réfraction, calibration PMMA): Mₚ = 10 600 g.mol⁻¹; Mₙ = 25 400 g.mol⁻¹; Ð = 2,4.

### Exemple 2

### Synthèse d'un composé de formule :

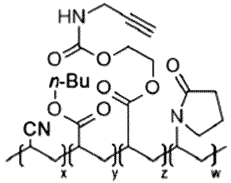

dans laquelle x = 1, y = 0,3, z = 0,2 et w = 0,1.

Un ballon tricol de 50mL est équipé d'un thermomètre, d'un réfrigérant et d'une agitation magnétique (300 tr/min). Il a ensuite été chargé avec 10 mL de dichlorométhane et et 1g d'un agent de liaison de formule ci-dessous, et plongé dans un bain-marie à température ambiante : 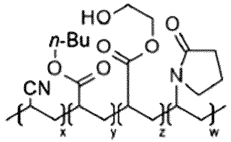 dans laquelle x = 1, y = 0,3, z = 0,2 et w = 0,1. Le CDI (365,88 mg ; 2,26mmoles ; 1,5 équivalents) a ensuite été additionné en une portion et la réaction s'est poursuivie à température ambiante durant 3h. La propargylamine (300 µL ; 4,72 mmoles ; 3 équivalents) a ensuite été additionnée en une portion et la réaction s'est poursuivie à température ambiante durant 18h. A ce stade, une RMN du proton du milieu réactionnel a montré une conversion totale. Le milieu réactionnel a été dilué dans 40 mL de dichlorométhane puis la phase organique a été lavée avec 3 portions de 50 mL d'eau distillée. Elle a ensuite été séchée sur sulfate de magnésium et évaporée sous pression réduite pour récupérer un solide jaune (1,31 g ; Quantitatif).

**¹H RMN** (acétone d₆, 400 MHz, 21°C): δ (ppm) = 6.69 (s(br), N***H***), 4.33 (s(br), -OC***H₂***C***H₂***O-), 4.15 (s(br), -OC***H₂***CH₂CH₂CH₃), 3.95 (s(br), -C***H₂***C≡CH), 3.58 - 2.53 (m, C***H_{chaîne principale}***)*,* 2.68 (s(br), -CH₂C≡C***H***), 2.40 - 1.75 (m, *C**H_{2 chaîne principale}***)*,* 1.66 (s(br), -OCH₂C***H₂***CH₂CH₃), 1.42 (s(br), -OCH₂CH₂C***H₂***CH₃), 0.95 (s(br), -OCH₂CH₂CH₂C***H₃***).

**ATR-FTIR** : ν (cm⁻¹) = 3368 (w), 3282 (w), 2960 (m), 2931 (m), 2874(w), 2242(w), 1720(s), 1677(m), 1520(m), 1452(m), 1239(s), 1168(s), 1058(m), 944(w), 776(w), 652(m).

**SEC** (DMSO, Indice de réfraction, calibration PMMA): Mₚ = 12 600 g.mol⁻¹; Mₙ = 7 000 g.mol⁻¹; Ð = 1,8.

## Revendications

1. Procédé de synthèse d'un composé de formule (I) : 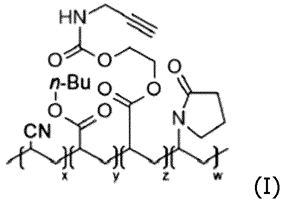
dans laquelle x = 1 ; 0 < w ≤ 1 ; 0 < y ≤ 1 et 0 < z ≤ 1 ;
le procédé comprenant :
a) la réaction d'un composé de formule (II) : 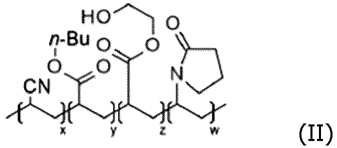
dans laquelle, w, x, y et z sont tels que définis ci-dessus pour la formule (I),
avec un excès de carbonyldiimidazole, éventuellement en présence d'un solvant ;
b) la réaction du composé obtenu à l'étape a) avec un excès de propargylamine, éventuellement en présence d'un solvant.

2. Procédé selon la revendication 1, dans lequel l'étape a) est mise en œuvre à une température comprise entre la température ambiante et 40°C.

3. Procédé selon la revendication 1 ou la revendication 2, dans lequel la durée de l'étape a) est comprise entre 15 minutes et 10h.

4. Procédé selon l'une quelconque des revendications 1 à 3, dans lequel l'étape a) est mise en œuvre en présence d'un solvant.

5. Procédé selon l'une quelconque des revendications 1 à 4, dans lequel l'étape b) est mise en œuvre à une température comprise entre la température ambiante et 40°C.

6. Procédé selon l'une quelconque des revendications 1 à 5, dans lequel la durée de l'étape b) est comprise entre 1h et 48h.

7. Procédé selon l'une quelconque des revendications 1 à 6, dans lequel l'étape b) est mise en œuvre en présence d'un solvant.

8. Procédé selon l'une quelconque des revendications 1 à 7, dans lequel les étapes a) et b) sont mises en œuvre d'un solvant, et le solvant utilisé à l'étape a) et le solvant utilisé à l'étape b) sont identiques.

9. Composé de formule (I) susceptible d'être obtenu par le procédé de l'un quelconque des revendications 1 à 8 : 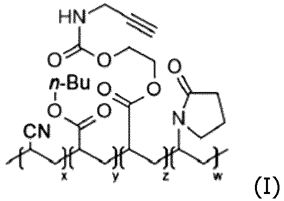 formule dans laquelle x = 1 ; 0 < w ≤ 1 ; 0 < y ≤ 1 et 0 < z ≤ 1.

## Patentansprüche

1. Verfahren zur Synthese einer Verbindung der Formel (I): 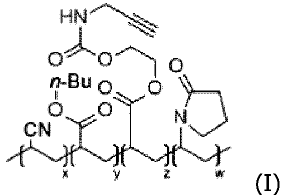
wobei x = 1 ; 0 < w ≤ 1 ; 0 < y ≤ 1 und 0 < z ≤ 1 ;
wobei das Verfahren umfasst:
a) die Umsetzung einer Verbindung der Formel (II): 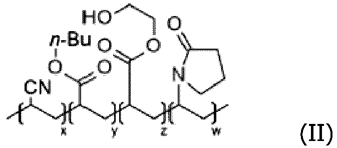
wobei w, x, y und z wie oben für die Formel (I) definiert sind,
mit einem Überschuss an Carbonyldiimidazol, gegebenenfalls in Gegenwart eines Lösungsmittels;
b) die Umsetzung der in Schritt a) erhaltenen Verbindung mit einem Überschuss an Propargylamin, gegebenenfalls in Gegenwart eines Lösungsmittels.

2. Verfahren nach Anspruch 1, wobei Schritt a) bei einer Temperatur zwischen Raumtemperatur und 40 °C durchgeführt wird.

3. Verfahren nach Anspruch 1 oder Anspruch 2, wobei die Dauer von Schritt a) zwischen 15 Minuten und 10 Stunden liegt.

4. Verfahren nach einem der Ansprüche 1 bis 3, wobei der Schritt a) in Gegenwart eines Lösungsmittels durchgeführt wird.

5. Verfahren nach einem der Ansprüche 1 bis 4, wobei der Schritt b) bei einer Temperatur zwischen Raumtemperatur und 40 °C durchgeführt wird.

6. Verfahren nach einem der Ansprüche 1 bis 5, wobei die Dauer des Schritts b) zwischen 1 Stunde und 48 Stunden liegt.

7. Verfahren nach einem der Ansprüche 1 bis 6, wobei der Schritt b) in Gegenwart eines Lösungsmittels durchgeführt wird.

8. Verfahren nach einem der Ansprüche 1 bis 7, wobei die Schritte a) und b) in Gegenwart eines Lösungsmittels durchgeführt werden und das in Schritt a) verwendete Lösungsmittel und das in Schritt b) verwendete Lösungsmittel identisch sind.

9. Verbindung der Formel (I), die durch das Verfahren gemäß einem der Ansprüche 1 bis 8 erhältlich ist: 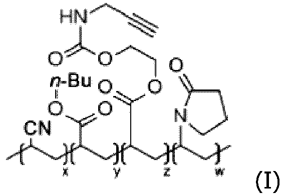 Formel, in der x = 1 ; 0 < w ≤ 1 ; 0 < y ≤ 1 und 0 < z ≤ 1.

## Claims

1. A process for synthesizing a compound of formula (I): 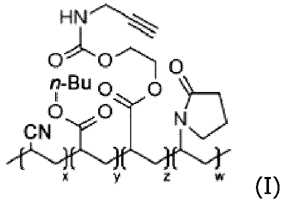
In which x =1 ; 0 < w ≤ 1 ; 0 < y ≤ 1 and 0 < z ≤ 1 ;
the process comprising:
a) reacting a compound of formula (II): 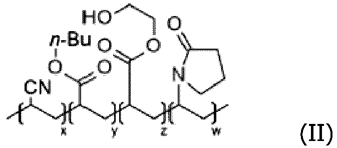
in which, w, x, y and z are as defined above for formula (I),
with an excess of carbonyldiimidazole, optionally in the presence of a solvent;
b) reacting the compound obtained in step a) with an excess of propargylamine, optionally in the presence of a solvent.

2. The process of claim 1, wherein step a) is performed at a temperature between room temperature and 40°C.

3. The process of claim 1 or claim 2, wherein the duration of step a) is between 15 minutes and 10h.

4. The process of any one of claims 1 to 3, wherein step a) is performed in the presence of a solvent.

5. The process of any one of claims 1 to 4, wherein step b) is performed at a temperature between room temperature and 40°C.

6. The process of any one of claims 1 to 5, wherein the duration of b) is between 1h and 48h.

7. The process of any one of claims 1 to 6, wherein step b) is performed in the presence of a solvent.

8. The process of any one of claims 1 to 7, wherein steps a) and b) are performed in the presence of a solvent, and the solvent used in step a) is identical to the solvent used in step b).

9. A compound of formula (I) obtainable by the process of any one of claims 1 to 8: 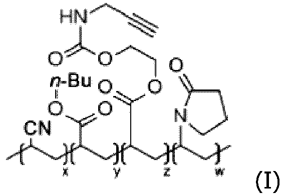 in which formula x = 1 ; 0 < w ≤ 1 ; 0 < y ≤ 1 and 0 < z ≤ 1.
